# EUROPÄISCHE PATENTSCHRIFT

(11) **EP 2 104 663 B1**
(45) Veröffentlichungstag und Bekanntmachung des Hinweises auf die Patenterteilung: **15.07.2015**
(21) Anmeldenummer: 08701028.6
(22) Anmeldetag: 09.01.2008
(51) Int. Cl.: C07D 231/16

(54) **VERFAHREN ZUM HERSTELLEN VON SUBSTITUIERTEN PYRAZOLCARBONSÄURECHLORIDEN**
METHOD FOR PRODUCING SUBSTITUTED PYRAZOLECARBOXYLIC ACID CHLORIDES
PROCÉDÉ DE PRÉPARATION DE CHLORURES D'ACIDE PYRAZOLCARBOXYLIQUE SUBSTITUÉS

(30) Priorität: 18.01.2007 DE 102007002674
(43) Veröffentlichungstag der Anmeldung: 30.09.2009
(73) Patentinhaber: Bayer Intellectual Property GmbH, 40789 Monheim am Rhein (DE)
(72) Erfinder: LUI, Norbert, 51519 Odenthal (DE); HEINRICH, Jens-Dietmar, 51399 Burscheid (DE); STRAUB, Alexander, 42117 Wuppertal (DE); WOLLNER, Thomas, 50825 Köln (DE); FORD, Mark, James, 61389 Schmitten (DE)
(74) Vertreter: BIP Patents
(86) Internationale Anmeldenummer: PCT/EP2008/000091
(87) Internationale Veröffentlichungsnummer: WO 2008/086962

(56) Entgegenhaltungen:
- EP-A- 0 776 889
- WO-A-93/11117
- JP-A- 2 085 257
- JP-A- 7 010 845

## Beschreibung

Die vorliegende Erfindung betrifft ein Verfahren zum Herstellen von substituierten Pyrazolsäurechloriden durch Chlorierung von Aldehyden der Formel (II) unter radikalischen Bedingungen.

Pyrazolsäurechloride sind wichtige Bausteine zur Herstellung von Pflanzenschutzwirkstoffen.

Pyrazolsäurechloride stellt man üblicherweise durch Umsetzung von Carbonsäuren mit einem Chlorierungsmittel dar. Ein Vorteil dieses Verfahrens beruht darauf, dass die entsprechenden Carbonsäuren einfach zugänglich und damit im technischen Maßstab verfügbar sind. Diese Vorrausetzung ist bei der Herstellung der substituierten Pyrazolsäurechloriden nicht gegeben, da die entsprechenden Carbonsäuren nicht leicht zugänglich sind.

So werden die Pyrazol-4-carbonsäuren aus Pyrazol-4-carboxyaldehyden (J.L. Huppatz : Aust. J. Chem. 36, 135 - 147 (1983)) mit Kaliumpermanganat gemäß Schema I hergestellt. Ein Problem bei der technischen Umsetzung dieser Oxidation ist die Bildung von großen Mengen an Mangandioxyd.

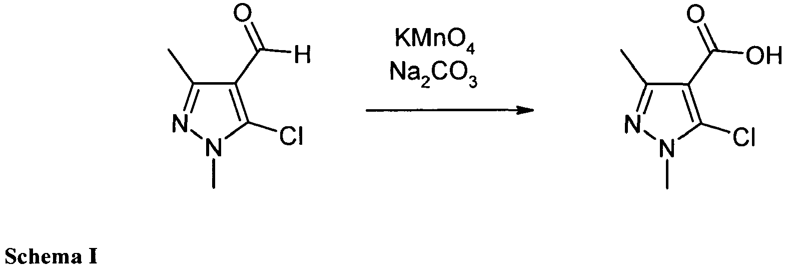

JP 7070076 beschreibt die Herstellung von Pyrazol-4-carbonsäure durch Reaktion des Pyrazol-4-carbaldehyds mit Wasserstoffperoxyd und Natriumchlorid im wässrigen System unter sauren Bedingungen und in Gegenwart oder Abwesenheit eines Phasentransferkatalysators. Die Raum-Zeit-Ausbeuten sind gering und das Verfahren ist aufgrund von Wasserstoffperoxyd sicherheitstechnologisch schwierig.

Aus der Pyrazol-4-carbonsäure muss dann in einem zweiten Schritt mit Hilfe eines Chlorierungsmittels, wie z.B. Thionylchlorid, das Pyrazolcarbonsäurechlorid hergestellt werden.

Im Hinblick auf die vorstehend geschilderten Nachteile und Probleme besteht der Bedarf ein Verfahren bereitzustellen, das in einem Schritt ausgehend von substituierten Pyrazolcarboxyaldehyden die entsprechenden substituierten Carbonsäurechloride mit hohe Ausbeuten und hoher Selektivität zugänglich macht.

Gegenstand der vorliegenden Erfindung ist daher ein Verfahren zum Herstellen von substituierten Pyrazolcarbonsäurechloriden der allgemeinen Formel (I) 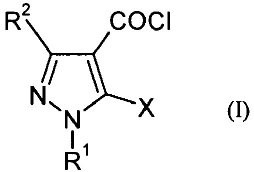 in welcher
- R¹: für Wasserstoff, Alkyl, Alkoxyalkyl, unsubstituiertes oder substituiertes Aryl, unsubstituiertes oder substituiertes Aralkyl, Alkoxyalkyl, unsubstituiertes oder substituiertes Aryl, unsubstituiertes oder substituiertes Alkaryl, unsubstituiertes oder substituiertes Heteroaryl und Halogenalkyl steht,
- R²: für Wasserstoff, Halogen, Alkyl, Alkoxyalkyl, unsubstituiertes oder substituiertes Aryl, unsubstituiertes oder substituiertes Aralkyl, unsubstituiertes oder substituiertes Heteroaryl und Halogenalkyl steht,
- X: für Fluor, Chlor oder Brom steht,
dadurch gekennzeichnet, dass man
Aldehyde der Formel (II) 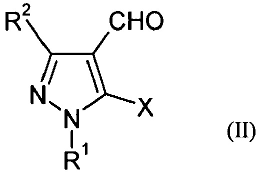 in welcher R¹, R², und X die oben genannte Bedeutung hat, mit einem Chlorierungsmittel unter radikalischen Bedingungen umsetzt.

### Allgemeine Definitionen

Im Zusammenhang mit der vorliegenden Erfindung umfasst der Begriff Halogene (X), soweit nicht anders definiert, solche Elemente, die ausgewählt sind aus der Gruppe bestehend aus Fluor, Chlor, Brom und Iod, wobei Fluor, Chlor und Brom bevorzugt und Fluor und Chlor besonders bevorzugt verwendet werden.

Gegebenenfalls substituierte Reste können einfach oder mehrfach substituiert sein, wobei bei Mehrfachsubstitutionen die Substituenten gleich oder verschieden sein können.

Mit einem oder mehreren Halogenatomen substituierte Alkylgruppen sind beispielsweise ausgewählt aus Trifluormethyl (CF₃), Difluormethyl (CHF₂), CF₃CH₂, ClCH₂, CF₃CCl₂.

Alkyl-Gruppen sind im Zusammenhang mit der vorliegenden Erfindung, soweit nicht abweichend definiert, lineare, verzweigte oder ringförmige Kohlenwasserstoff-Gruppen, die optional eine, zwei oder mehrere einfache oder zweifache Unsättigungen oder ein, zwei oder mehrere Heteroatome, die ausgewählt sind aus O, N, P und S aufweisen können. Außerdem können die erfindungsgemäßen Alkyl-Reste optional durch weitere Gruppen substituiert sein, die ausgewählt sind aus-R', Halogen- (-X), Alkoxy- (-OR'), Thioether- oder Mercapto- (-SR'), Amino- (-NR'₂), Silyl-(-SiR'₃), Carboxyl- (-COOR'), Cyan- (-CN), Acyl- (-(C=O)R') und Amid-Gruppen (-CONR₂'), wobei R' Wasserstoff oder eine C₁₋₁₂-Alkyl-Gruppe, vorzugsweise C₂₋₁₀-Alkyl-Gruppe, besonders bevorzugt C₃₋₈-Alkyl-Gruppe sein kann.

Die Definition C₁₋₁₂-Alkyl umfasst den größten hierin definierten Bereich für einen Alkyl-Rest. Im Einzelnen umfasst diese Definition beispielsweise die Bedeutungen Methyl, Ethyl, n-, iso-Propyl, n-, iso-, sec- und t-Butyl, n-Pentyl, n-Hexyl, 1,3-Dimethylbutyl, 3,3-Dimethylbutyl, n-Heptyl, n-Nonyl, n-Decyl, n-Undecyl, n-Dodecyl.

Aryl-Reste sind im Zusammenhang mit der vorliegenden Erfindung, soweit nicht abweichend definiert, aromatische Kohlenwasserstoff-Reste, die ein, zwei oder mehrere Heteroatome, die ausgewählt sind aus O, N, P und S aufweisen können und optional durch weitere Gruppen substituiert sein können, die ausgewählt sind aus -R', Halogen- (-X), Alkoxy- (-OR'), Thioether- oder Mercapto- (-SR'), Amino- (-NR'₂), Silyl- (-SiR'₃), Carboxyl- (-COOR'), Cyan- (-CN), Acyl-(-(C=O)R') und Amid-Gruppen (-CONR₂'), wobei R' Wasserstoff oder eine C₁₋₁₂-Alkyl-Gruppe, vorzugsweise C₂₋₁₀-Alkyl-Gruppe, besonders bevorzugt C₃₋₈-Alkyl-Gruppe sein kann.

Die Definition C₅₋₁₈-Aryl umfasst den größten hierin definierten Bereich für einen Aryl-Rest mit 5 bis 18 Gerüstkohlenstoffatomen. Im Einzelnen umfasst diese Definition beispielsweise die Bedeutungen Cyclopentadienyl, Phenyl, Cycloheptatrienyl, Cyclooctatetraenyl, Naphthyl und Anthracenyl.

Heteroaryl-Reste sind im Zusammenhang mit der vorliegenden Erfindung, soweit nicht abweichend definiert, aromatische Kohlenwasserstoff-Reste, die ein, zwei, drei oder mehrere Heteroatome, die ausgewählt sind aus O, N, P und S aufweisen und optional durch weitere Gruppen substituiert sein können, die ausgewählt sind aus -R', Halogen- (-X), Alkoxy- (-OR'), Thioether- oder Mercapto- (-SR'), Amino- (-NR'₂), Silyl- (-SiR'₃), Carboxyl- (-COOR'), Cyan-(-CN), Acyl- (-(C=O)R') und Amid-Gruppen (-CONR₂'), wobei R' Wasserstoff oder eine C₁₋₁₂-Alkyl-Gruppe, vorzugsweise C₂₋₁₀-Alkyl-Gruppe, besonders bevorzugt C₃₋₈-Alkyl-Gruppe sein kann.

Die Definition C₅₋₁₈-Heteroaryl umfasst den größten hierin definierten Bereich für einen Aryl-Rest mit 5 bis 18 Gerüstkohlenstoffatomen, wobei die Gerüstkohlenstoffatome gegen die zuvor genannten Heteroatome ersetzt sein können.. Im Einzelnen umfasst diese Definition beispielsweise die Bedeutungen 2-Furyl, 3-Furyl, 2-Thienyl, 3-Thienyl, 2-Pyrrolyl, 3-Pyrrolyl, 3-Isoxazolyl, 4-Isoxazolyl, 5-Isoxazolyl, 3-Isothiazolyl, 4-Isothiazolyl, 5-Isothiazolyl, 3-Pyrazolyl, 4-Pyrazolyl, 5-Pyrazolyl, 2-Oxazolyl, 4-Oxazolyl, 5-Oxazolyl, 2-Thiazolyl, 4-Thiazolyl, 5-Thiazolyl, 2-Imidazolyl, 4-Imidazolyl, 1,2,4-Oxadiazol-3-yl, 1,2,4-Oxadiazol-5-yl, 1,2,4-Thiadiazol-3-yl, 1,2,4-Thiadiazol-5-yl, 1,2,4-Triazol-3-yl, 1,3,4-Oxadiazol-2-yl, 1,3,4-Thiadiazol-2-yl und 1,3,4-Triazol-2-yl; 1-Pyrrolyl, 1-Pyrazolyl, 1,2,4-Triazol-1-yl, 1-Imidazolyl, 1,2,3-Triazol-1-yl, 1,3,4-Triazol-1-yl; 3-Pyridazinyl, 4-Pyridazinyl, 2-Pyrimidinyl, 4-Pyrimidinyl, 5-Pyrimidinyl, 2-Pyrazinyl, 1,3,5-Triazin-2-yl und 1,2,4-Triazin-3-yl.

Arylalkyl-Reste (Aralkyl-Reste) sind im Zusammenhang mit der vorliegenden Erfindung, soweit nicht abweichend definiert, durch Aryl-Gruppen substituierte Alkyl-Reste, die eine C₁₋₈-Alkylenkette aufweisen können und im Arylgerüst oder der Alkylenkette durch eines oder mehrere Heteroatome, die ausgewählt sind aus O, N, P und S und optional durch weitere Gruppen substituiert sein können, die ausgewählt sind aus -R', Halogen- (-X), Alkoxy- (-OR'), Thioether- oder Mercapto- (-SR'), Amino- (-NR'₂), Silyl- (-SiR'₃), Carboxyl- (-COOR'), Cyan- (-CN), Acyl-(-(C=O)R') und Amid-Gruppen (-CONR₂'), wobei R' Wasserstoff oder eine C₁₋₁₂-Alkyl-Gruppe, vorzugsweise C₂₋₁₀-Alkyl-Gruppe, besonders bevorzugt C₃₋₈-Alkyl-Gruppe sein kann.

Die Definition C₇₋₁₉-Aralkyl-Rest umfasst den größten hierin definierten Bereich für einen Arylalkyl-Rest mit insgesamt 7 bis 19 Kohlenstoffatomen in Gerüst und Alkylenkette. Im Einzelnen umfasst diese Definition beispielsweise die Bedeutungen Benzyl und Phenylethyl.

Alkylaryl-Reste (Alkaryl-Reste) sind im Zusammenhang mit der vorliegenden Erfindung, soweit nicht abweichend definiert, durch Alkyl-Gruppen substituierte Aryl-Reste, die eine C₁₋₈-Alkylenkette aufweisen können und im Arylgerüst oder der Alkylenkette durch eines oder mehrere Heteroatome, die ausgewählt sind aus O, N, P und S und optional durch weitere Gruppen substituiert sein können, die ausgewählt sind aus -R', Halogen- (-X), Alkoxy- (-OR'), Thioether- oder Mercapto- (-SR'), Amino- (-NR'₂), Silyl- (-SiR'₃), Carboxyl- (-COOR'), Cyan- (-CN), Acyl-(-(C=O)R') und Amid-Gruppen (-CONR₂'), wobei R' Wasserstoff oder eine C₁₋₁₂-Alkyl-Gruppe, vorzugsweise C₂₋₁₀-Alkyl-Gruppe, besonders bevorzugt C₃₋₈-Alkyl-Gruppe sein kann.

Die Definition C₇₋₁₉Alkylaryl-Rest umfasst den größten hierin definierten Bereich für einen Alkylaryl-Rest mit insgesamt 7 bis 19 Kohlenstoffatomen in Gerüst und Alkylenkette. Im Einzelnen umfasst diese Definition beispielsweise die Bedeutungen Tolyl-, 2,3-, 2,4-, 2,5-, 2,6-, 3,4- oder 3,5-Dimethylphenyl.

Nach dem erfindungsgemäßen Verfahren lassen sich Säurechloride der Formel (I) in guten Ausbeuten in einer Stufe aus den entsprechenden Aldehyden der Formel (II) herstellen. Dies ist überraschend, da die Chlorierung von Verbindungen der Formel (II) mit Chlorierungsmitteln in Abwesenheit eines Radikalstarters nach einem vollständig anderer Reaktionsverlauf erfolgt, welches in Schema II dargestellt ist.

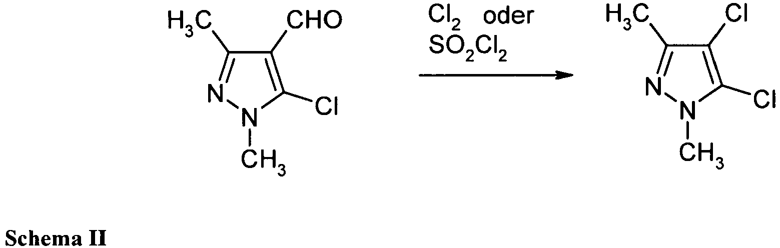

Bevorzugt ist die Durchführung des erfindungsgemäßen Verfahrens unter Verwendung des Aldehyds der Formel (II), in denen die angegeben Reste jeweils folgende Bedeutungen haben. Die bevorzugten, besonders bevorzugten und ganz besonders bevorzugten Bedeutungen gelten für alle Verbindungen, in denen die jeweiligen Reste vorkommen.

In Formel (I) und (II) steht
- R¹: für Wasserstoff, C₁₋₁₂-Alkyl, C₁₋₄-Alkoxy-C₁₋₁₂-alkyl, unsubstituiertes oder substituiertes C₅₋₁₈-Aryl, unsubstituiertes oder substituiertes C₅₋₁₉-Aralkyl, unsubstituiertes oder substituiertes C₅₋₁₉-Alkaryl, unsubstituiertes oder substituiertes C₅₋₁₈-Heteroaryl und C₁₋₁₂-Halogenalkyl mit 1 bis 5 Halogenatomen; bevorzugt für C₁ bis C₃-Alkyl, besonders bevorzugt für Methyl
- R²: für Wasserstoff, Halogen, C₁₋₁₂-Alkyl, C₁₋₄-Alkoxy-C₁₋₁₂-alkyl, unsubstituiertes oder substituiertes Aryl, unsubstituiertes oder substituiertes C₅₋₁₉-Aralkyl, unsubstituiertes oder substituiertes C₅₋₁₉-Alkaryl, unsubstituiertes oder substituiertes C₅₋₁₈-Heteroaryl und C₁₋₁₂-Halogenalkyl mit 1 bis 5 Halogenatomen; bevorzugt für Wasserstoff, Halogen, C₁₋₅-Alkyl, und C₁₋₅-Halogenalkyl, besonders bevorzugt Methyl.
- X: steht für Fluor, Chlor oder Brom, bevorzugt für Fluor und Chlor, besonders bevorzugt für Chlor.

Die bei der Durchführung des erfindungsgemäßen Verfahrens als Ausgangsstoffe zu verwendenden Aldehyde der Formel (II) sind im Stand der Technik vorbeschrieben oder können nach bekannten Verfahren, beispielsweise durch Vilsmeier-Haack Reaktion mit DMF und POCl₃, erhalten werden.

Man setzt üblicherweise den substituierten Aldehyd der Formel (II) und das Chlorierungsmittel im Molverhältnis 1 : 3 bis 3 : 1, bevorzugt 1 : 1,4 bis 1 : 1.

Als Chlorierungsmittel kann man Chlor, oder ein chlorabgebendes Agens einsetzten. Die Reaktion kann ggf. in Gegenwart eines inerten Verdünnungsgases wie z.B. Stickstoff, Kohlendioxyd oder Edelgase durchgeführt werden. Geeignet als Chlorierungsmittel, ohne Anspruch auf Vollständigkeit zu erheben, sind beispielsweise Cl₂, SO₂Cl₂, SOCl₂, N-Chlorsuccinimid oder ein Gemisch derselben. Man setzt bevorzugt Cl₂, SO₂Cl₂, oder ein Gemisch derselben als Chlorierungsmittel ein. Besonders bevorzugt ist Cl₂ als Chlorierungsmittel.

Gemäß der vorliegenden Erfindung erfolgt die Chlorierung unter radikalischen Bedingungen. Voraussetzung dafür ist die Bildung von Chlorradikalen.

Die Erzeugung von Radikalen kann durch unterschiedliche im Stand der Technik vorbeschriebene und dem Fachmann geläufige Methoden erfolgen.

Radikale können prinzipiell auf drei Arten erzeugt werden: (a) durch homolytischen Bindungsbruch, (b) durch Reaktion mit anderen Radikalen und (c) durch Einelektronen-Übertragung (single electron transfer). Bei der Erzeugung von Radikalen durch Homolyse werden labile Bindungen thermisch, photochemisch oder radiolytisch gespalten.

Im Zusammenhang mit der vorliegenden Erfindung werden die radikalischen Bedingungen vorzugsweise durch die Umsetzung der substituierten Aldehyde mit dem Chlorierungsmittel in Anwesenheit eines Radikalstarters oder durch Bestrahlung geschaffen.

Es ist bekannt, dass organische Peroxide oder Azoverbindungen unter der Einwirkung von Wärme und/oder Licht in Radikale zerfallen, die die radikalische Chlorierung initiieren.

Beispiele, ohne Anspruch auf Vollständigkeit, geeigneter Peroxide und Azoverbindungen sind tert.-Butylhydroperoxyd, Dibenzoylperoxid, Di(4-tert-butylcyclohexyl)peroxydicarbonat, 2,2'-Azobis(isobutyronitril), Dimethyl-2,2'-azobis(isobutyrat), 2,2'-Azobis(2,4-dimethylvaleronitril), Di-(2-ethylhexyl)peroxydicarbonat, tert-Butylperoxypivalat, tert.-Amylperoxy-2-ethylhexanoat, tert-Butylperoxy-2-ethylhexanoat.

Bevorzugt setzt man folgende Radikalstarter ein: 2,2'-Azobis(isobutyronitril), 2,2'-Azobis(2,4-dimethylvaleronitril), Di(2-ethylhexyl)peroxydicarbonat, tert-Amylperoxy-2-ethylhexanoat, tert-Butyl peroxy-2-ethylhexanoat. Man setzt den Radikalstarter üblicherweise in einer Menge von 0,01 bis 1 Mol-%, vorzugsweise 0,1 bis 0,5 Mol-% bezogen auf den Aldehyd der Formel (II) ein.

Gemäß einer alternativen erfindungsgemäßen Ausführungsform können die Radikale können auch durch Bestrahlung mit elektromagnetischer Strahlung, vorzugsweise mit Licht oder Röntgenstrahlung erzeugt werden. Verwendet werden können hierzu z.B. Quecksilberlampen.

Die Umsetzung der substituierten Aldehyde (II) mit dem Chlorierungsmittel erfolgt üblicherweise in Gegenwart eines Verdünnungsmittels, das sich unter den vorherrschenden Reaktionsbedingungen inert verhält. Man kann als Verdünnungsmittel beispielsweise einfach oder mehrfach chlorierte aliphatische oder aromatische Kohlenwasserstoffe oder Gemische derselben einsetzen. Beispiele für geeignete Verdünnungsmittel sind Chlorbenzol, Dichlorbenzole, Trichlorbenzole, Chlortoluole, Chlorbenzotrifluoride, Methylenchlorid, Dichlorethan, Chloroform, Tetrachlorkohlenstoff. Bevorzugte Verdünnungsmittel sind Chlorbenzol, 1,2-Dichlorbenzol, 1,3-Dichlorbenzol, 1,4-Dichlorbenzol, 1,2,3-Trichlorbenzol, 1,2,4-Trichlorbenzol, 4-Chlortrifluormethylbenzol, 1,3,5-Trichlorbenzol, 2-Chlortoluol, 3-Chlortoluol, 4-Chlortoluol oder ein Gemisch derselben. Besonders bevorzugt verwendet man Chlorbenzol.

Man setzt das Verdünnungsmittel üblicherweise im Verhältnis 20 : 1 bis 1: 20 , bevorzugt 10: 1 bis 1: 10 bezogen auf den substituierten Aldehyd (II) ein.

Die Reaktion des substituierten Aldehyds (II) mit dem Chlorierungsmittel kann beispielsweise bei -10 bis 130 °C durchgeführt werden, bevorzugt sind Temperaturen von 0 - 90°C.

Der Reaktionsmischung kann ggf. ein Säurefänger zugesetzt werden. Als Säurefänger können beispielsweise Kaliumcarbonat, Natriumcarbonat, Calciumcarbonat, Kaliumhydrogencarbonat, Natriumhydrogencarbonat, Natriumhydroxyd, Kaliumhydroxyd verwendet werden. Diese Säurefänger können auch als wässrige Lösung eingesetzt werden.

Die Isolierung der substituierten Pyrazolsäurechloride (I) kann durch übliche Aufarbeitungsverfahren erfolgen. Hierbei sind die Schmelz- und Siedepunkte der Produkte entscheidend und bestimmen, ob das Produkt destilliert oder durch Filtration isoliert wird. Die destillative Aufarbeitung ist bevorzugt.

Das erfindungsgemäße Verfahren lässt sich diskontinuierlich oder kontinuierlich durchführen. Das Verfahren lässt sich unter Normaldruck, Unter- oder Überdruck durchführen.

Das erfindungsgemäße Verfahren zum Herstellen von substituierter Pyrazolsäurechloride wird in den nachstehenden Beispielen beschrieben, welche die obige Beschreibung weiter illustrieren. Die Beispiele sind jedoch nicht in einschränkender Weise zu interpretieren.

### Herstellungsbeispiele:

### Vergleichsbeispiel: Chlorierung, ohne Radikale

300 g einer 22,7 % igen Lösung von 5-Chlor-1,3-dimethyl-1H-pyrazol-4-carbaldehyd in 1,2-Dichlorbenzol werden bei 80 °C innerhalb 120 Minuten 58 g Sulfurylchlorid zugetropft. Nach Beendigung der Zudosierung wird die Reaktionslösung weitere 3 Stunden gerührt. Die Reaktionslösung wird auf 20°C abgekühlt und die Lösung wird per GC analysiert. Es ist quantitativ das 4,5-Dichlor-1,3-dimethyl-1H-pyrazol entstanden.

### Beispiel 1

Zu einer Lösung von 48,5 g 5-Chlor-1,3-dimethyl-1H-pyrazol-4-carbaldehyd in 230 ml Chlorbenzol werden 36 g Natriumcarbonat zugesetzt. Anschließend wird bei 60 °C 3,9 g 2,2-Azoisobutyronitril (AIBN) zugesetzt und innerhalb 4 Stunden 24 g Chlorgas eingeleitet und weitere 6 g AIBN zugesetzt. Nach Beendigung der Gaseinleitung wird die Reaktionslösung weitere 30 Minuten gerührt. Der Feststoff wird abfiltriert und mit wenig Chlorbenzol gewaschen. Nach Entfernung des Lösungsmittels erhält man 5-Chlor-1,3-dimethyl-1H-pyrazol-4-carbonsäurechlorid in einer Ausbeute von 74 %.

### Beispiel 2

Zu einer Lösung von 48 g 5-Chlor-1,3-dimethyl-1H-pyrazol-4-carbaldehyd in 230 ml Chlorbenzol werden 36,5 g Natriumcarbonat zugesetzt. Anschließend wird bei 20 °C 1,2 g Bis(tert.-Butylcyclohexyl)-peroxicarbonat zugesetzt und auf 40°C erwärmt. Innerhalb 4 Stunden werden 24 g Chlorgas eingeleitet. Nach Beendigung der Gaseinleitung wird die Reaktionslösung weitere 30 Minuten bei 40°C gerührt. Der Feststoff wird abfiltriert und mit wenig Chlorbenzol gewaschen. Nach Entfernung des Lösungsmittels erhält man in einer Ausbeute von 68 % 5-Chlor-1,3-dimethyl-1H-pyrazol-4-carbonsäurech lorid.

### Beispiel 3

Zu einer Lösung von 48 g 5-Chlor-1,3-dimethyl-1H-pyrazol-4-carbaldehyd in 230 ml Chlorbenzol unter Schutzgas werden 51,8 gl Kaliumcarbonat zugesetzt. Anschließend wird unter Belichtung mit einer Quecksilberlampe bei 20 °C innerhalb 4 Stunden 24 g Chlorgas eingeleitet. Nach Beendigung der Gaseinleitung wird die Reaktionslösung weitere 20 Minuten bei 20°C unter Belichtung gerührt. Der Feststoff wird abfiltriert und mit wenig Chlorbenzol gewaschen. Nach Entfernung des Lösungsmittels erhält man in einer Ausbeute von 95 % 5-Chlor-1,3-dimethyl-1H-pyrazol-4-carbonsäurechlorid.

### Beispiel 4

Zu einer Lösung von 48 g 5-Chlor-1,3-dimethyl-1H-pyrazol-4-carbaldehyd in 195 ml Dichlorbenzol unter Schutzgas werden 39,8 g Natriumcarbonat zugesetzt. Anschließend wird unter Belichtung mit einer Quecksilberlampe bei 20 °C innerhalb 3,5 Stunden 24 g Chlorgas eingeleitet. Nach Beendigung der Gaseinleitung wird die Reaktionslösung weitere 20 Minuten bei 20°C unter Belichtung gerührt. Der Feststoff wird abfiltriert und mit wenig Dichlorbenzol gewaschen. Nach Entfernung des Lösungsmittels erhält man in einer Ausbeute von 87 % 5-Chlor-1,3-dimethyl-1H-pyrazol-4-carbonsäurechlorid.

### Beispiel 5

Zu einer Lösung von 48 g 5-Chlor-1,3-dimethyl-1H-pyrazol-4-carbaldehyd in 230 ml Chlorbenzol unter Schutzgas wird unter Belichtung mit einer Quecksilberlampe bei 30 °C innerhalb 4 Stunden 19,2 g Chlorgas eingeleitet. Nach Beendigung der Gaseinleitung wird die Reaktionslösung weitere 20 Minuten bei 30 °C unter Belichtung gerührt. Die Reaktionslösung wird auf 5 °C abgekühlt und der Feststoff wird abfiltriert und mit wenig Chlorbenzol gewaschen. Nach Entfernung des Lösungsmittels erhält man 5-Chlor-1,3-dimethyl-1H-pyrazol-4-carbonsäurechlorid in einer Ausbeute von 87 %.

### Beispiel 6

300 g einer 22,7 %igen Lösung von 5-Chlor-1,3-dimethyl-1H-pyrazol-4-carbaldehyd in 1,2-Dichlorbenzol werden bei 80 °C innerhalb von 4 Stunden 68 g Sulfurylchlorid und 70,5 g einer 2,5 %igen Lösung von 2,2-Azoisobutyronitril (AIBN) simultan zugetropft. Nach Beendigung der Zudosierung wird die Reaktionslösung weitere 2 Stunden gerührt. Nach Entfernung des Lösungsmittels erhält man 5-Chlor-1,3-dimethyl-1H-pyrazol-4-carbonsäurechlorid in einer Ausbeute von 72 %.

### Beispiel 7

11 g einer 10 %igen Lösung von 5-Chlor-1,3-dimethyl-1H-pyrazol-4-carbaldehyd in 1,2-Dichlorbenzol bei 10°C vorgelegt und anschließend eine Lösung von 4,6 g Kaliumcarbonat in 20 ml Wasser zugesetzt. Bei 10 °C werden unter Belichtung mit einer Quecksilberlampe innerhalb von 1 Stunde 8 g Chlorgas eingeleitet. Nach Beendigung der Gaseinleitung wird die Reaktionslösung mit Wasser versetzt, die organische Phase abgetrennt und mit Wasser gewaschen. Nach Entfernung des Lösungsmittels erhält man 5-Chlor-l ,3-dimethyl-l H-pyrazol-4-carbonsäurechlorid in einer Ausbeute von 77 %.

## Patentansprüche

1. Verfahren zum Herstellen von substituierten Pyrazolcarbonsäurechloriden der allgemeinen Formel (I) 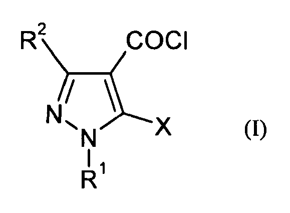 in welcher
R¹ für Wasserstoff, Alkyl, Alkoxyalkyl, unsubstituiertes oder substituiertes Aryl, unsubstituiertes oder substituiertes Aralkyl, unsubstituiertes oder substituiertes Heteroaryl und Halogenalkyl steht,
R² für Wasserstoff, Halogen, Alkyl, Alkoxyalkyl, unsubstituiertes oder substituiertes Aryl, unsubstituiertes oder substituiertes Aralkyl; unsubstituiertes oder substituiertes Heteroaryl und Halogenalkyl steht,
X für Fluor, Chlor oder Brom steht,
**dadurch gekennzeichnet, dass** man
Aldehyde der Formel (II) 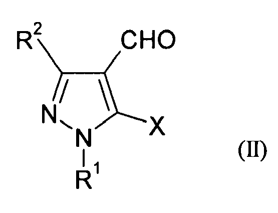 in welcher R¹, R², und X die oben genannte Bedeutung hat, mit einem Chlorierungsmittel unter radikalischen Bedingungen umsetzt.

2. Verfahren gemäß Anspruch 1, **dadurch gekennzeichnet, dass**
R¹ für C₁ bis C₃-Alkyl,
R² für Wasserstoff, Halogen, Alkyl, und Halogenalkyl und
X für Chlor, Brom oder Fluor steht.

3. Verfahren gemäß Anspruch 1 oder 2, **dadurch gekennzeichnet, dass**
R¹ für Methyl,
R² für Methyl und
X für Chlor oder Fluor steht.

4. Verfahren gemäß einem der Ansprüche 1 bis 3, wobei die radikalischen Bedingungen durch die Anwesenheit eines Radikalstarters oder durch Bestrahlung erzeugt werden.

5. Verfahren gemäß einem der Ansprüche 1 bis 4, wobei die Reaktion in Gegenwart eines Säurefängers durchgeführt wird.

6. Verfahren gemäß einem der Ansprüche 1 bis 5, wobei die Reaktion in Gegenwart einer wässrigen Base durchgeführt wird.

## Claims

1. Process for preparing substituted pyrazolecarbonyl chlorides of the general formula (I) 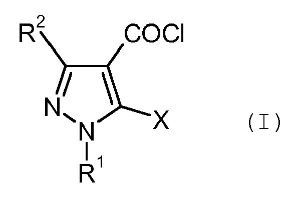 in which
R¹ is hydrogen, alkyl, alkoxyalkyl, unsubstituted or substituted aryl, unsubstituted or substituted aralkyl, unsubstituted or substituted heteroaryl and haloalkyl,
R² is hydrogen, halogen, alkyl, alkoxyalkyl, unsubstituted or substituted aryl, unsubstituted or substituted aralkyl, unsubstituted or substituted heteroaryl and haloalkyl,
X is fluorine, chlorine or bromine,
**characterized in that**
aldehydes of the formula (II) 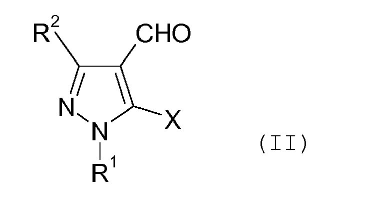 in which R¹, R² and X are each as defined above are reacted with a chlorinating agent under free-radical conditions.

2. Process according to Claim 1, **characterized in that**
R¹ is C₁- to C₃-alkyl,
R² is hydrogen, halogen, alkyl and haloalkyl, and
X is chlorine, bromine or fluorine.

3. Process according to Claim 1 or 2, **characterized in that**
R¹ is methyl,
R² is methyl and
X is chlorine or fluorine.

4. Process according to one of Claims 1 to 3, wherein the free-radical conditions are generated by the presence of a free-radical initiator or by irradiation.

5. Process according to one of Claims 1 to 4, wherein the reaction is performed in the presence of an acid scavenger.

6. Process according to one of Claims 1 to 5, wherein the reaction is performed in the presence of an aqueous base.

## Revendications

1. Procédé de fabrication de chlorures d'acides pyrazolecarboxyliques substitués de formule générale (I) 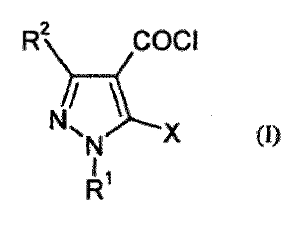 dans laquelle
R¹ représente hydrogène, alkyle, alcoxyalkyle, aryle non substitué ou substitué, aralkyle non substitué ou substitué, hétéroaryle non substitué ou substitué et halogénoalkyle,
R² représente hydrogène, halogène, alkyle, alcoxyalkyle, aryle non substitué ou substitué, aralkyle non substitué ou substitué, hétéroaryle non substitué ou substitué et halogénoalkyle,
X représente fluor, chlore ou brome,
**caractérisé en ce que**
des aldéhydes de formule (II) 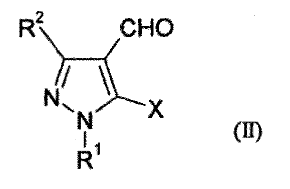 dans laquelle R¹, R² et X ont la signification indiquée précédemment, sont mis en réaction avec un agent de chloration dans des conditions radicalaires.

2. Procédé selon la revendication 1, **caractérisé en ce que**
R¹ représente alkyle en C₁ à C₃,
R² représente hydrogène, halogène, alkyle et halogénoalkyle, et
X représente chlore, brome ou fluor.

3. Procédé selon la revendication 1 ou 2, **caractérisé en ce que**
R¹ représente méthyle,
R² représente méthyle, et
X représente chlore ou fluor.

4. Procédé selon l'une quelconque des revendications 1 à 3, dans lequel les conditions radicalaires sont générées par la présence d'un démarreur radicalaire ou par exposition à un rayonnement.

5. Procédé selon l'une quelconque des revendications 1 à 4, dans lequel la réaction est réalisée en présence d'un capteur d'acides.

6. Procédé selon l'une quelconque des revendications 1 à 5, dans lequel la réaction est réalisée en présence d'une base aqueuse.
